# FASCICULE DE BREVET EUROPEEN

(11) **EP 1 245 577 B1**
(45) Date de publication et mention de la délivrance du brevet: **24.05.2006**
(21) Numéro de dépôt: 02290745.5
(22) Date de dépôt: 25.03.2002
(51) Int. Cl.: C08B 30/14, A01C 1/06, A61K 9/28, A61K 9/48, A23B 7/16, A23B 9/14, A23G 3/00, A61Q 11/00, A61K 9/00

(54) **Procédé de cuisson/séchage d'amidons riches en amylose**
Verfahren zum Kochen/Trocknen von amylosereichen Stärken
Process for cooking/drying of amylose rich starches

(30) Priorité: 26.03.2001 FR 0104023
(43) Date de publication de la demande: 02.10.2002
(73) Titulaire: Roquette Frères, 62136 Lestrem (FR)
(72) Inventeur: Carbone, Domenico, 15060 Pasturana (AL) (IT); Quettier, Claude, 59193 Erquinghem Lys (FR); Semino, Giovanni, 15063 Cassano Spinola (AL) (IT); Fossati, Ernesto, 15067 Novi Ligure (AL) (IT)
(74) Mandataire: Boulinguiez, Didier

(56) Documents cités:
- EP-A- 0 366 898
- WO-A-02/00205
- WO-A-99/09066
- US-A- 3 222 220
- US-A- 3 499 962
- US-A- 3 549 619
- US-A- 4 755 397
- DATABASE WPI Week 198139 Derwent Publications Ltd., London, GB; AN 1981-70928d XP002204661 & JP 56 101814 A (SHIBAOMI T), 14 août 1981 (1981-08-14)

## Description

L'invention a pour objet un nouveau procédé de préparation d'amidons riches en amylose prégélatinisés.

Elle a également pour objet les amidons obtenus selon ce procédé.

Les amidons prégélatinisés sont généralement préparés par des techniques thermiques, chimiques ou mécaniques susceptibles d'engendrer un gonflement des granules d'amidons de façon à ce qu'ils soient solubles dans l'eau froide.

Les techniques préférées sont l'atomisation, la cuisson sur tambour ou l'extrusion. L'autoclavage ou le chauffage indirect sur échangeur de chaleur sont des procédés de cuisson qui tendent à produire des dispersions colloïdales complexes consistant en granules intacts, fragmentés et gonflés.

Il est bien connu que les amidons riches en amylose, c'est à dire présentant plus de 50% en poids d'amylose, sont particulièrement difficiles à disperser et nécessitent de fortes températures de cuisson.

Le document US-A-3.086.890 décrit un procédé de préparation d'amylose prégélatinisée. Ce procédé consiste en un autoclavage à 191°C sous pression d'une solution d'amylose à 25% en poids de matière sèche au plus, puis un séchage sur tambour à 110-200°C. Les poudres obtenues sont amorphes et forment des gels irréversibles après dispersion. Leur densité apparente est élevée, c'est à dire généralement supérieure à 0,4 g/ml.

Le document US-A-3.607.394 décrit un procédé de prégélatinisation d'amidons présentant des teneurs en amylose inférieures à 60% en poids. Ce procédé consiste en une cuisson de l'amidon à 149°C suivie d'un séchage sur tambour, par atomisation ou autre type de moyen de séchage.

Le document EP 0.366.898 décrit un procédé couplé de cuisson/atomisation, permettant d'obtenir des produits amorphes, pratiquement non rétrogradés, et présentant une densité apparente supérieure à celle d'un même amidon ayant subi le même procédé mais en deux étapes distinctes, soit une densité apparente supérieure à 0.45 g/ml. La cuisson des amidons riches en amylose est effectuée sur jet-cooker à 143°C et l'atomisation est mise en oeuvre avec une température d'air entrant de 220°C. Ce document présente en figure 7 une comparaison des différents amidons prégélatinisés de l'art antérieur.

Partant de cet enseignement, la Demanderesse a alors trouvé, après de longs travaux de recherche, que l'on pouvait préparer un amidon riche en amylose prégélatinisé en mettant en oeuvre un procédé particulier conduisant à des produits de faible densité apparente tout en travaillant à des températures peu élevées, ce qu'aucune des techniques de l'art antérieur ne permettaient d'obtenir.

L'invention a donc pour objet un procédé de préparation d'amidon riche en amylose prégélatinisé caractérisé en ce qu'il comprend les étapes consistant :
- à former une suspension comprenant un amidon riche en amylose et de l'eau ;
- à soumettre ladite suspension à une cuisson par jet de vapeur à une température comprise entre 125 et 135°C de manière à obtenir une colle d'amidon riche en amylose prégélatinisé ;
- à sécher ledit amidon prégélatinisé sur tambour sécheur à une pression comprise entre 2 et 10 bars de manière à obtenir un amidon riche en amylose prégélatinisé.

L'amidon riche en amylose prégélatinisé obtenu peut être ensuite récupéré et broyé de manière à obtenir une poudre de granulométrie choisie en fonction des utilisations qui en seront faites par la suite.

On désigne par amidons riches en amylose au sens de la présente invention les amidons présentant une teneur en amylose supérieure ou égale à 50% en poids, et de préférence comprise entre 50 et 80% en poids, provenant de toutes origines végétales comme notamment le maïs, la pomme de terre, le manioc.

De préférence, le procédé selon l'invention met en oeuvre un amidon de maïs comme par exemple l'EURYLON® commercialisé par la Demanderesse.

L'invention s'applique également aux amidons riches en amylose modifiés par voie chimique ou physique. De préférence, on choisira l'amidon modifié dans le groupe constitué par les amidons acétylés, hydroxypropylés, carboxyméthylés, fluidifiés, et les octényl succinates d'amidon.

Selon une variante préférée du procédé selon l'invention, on choisira un amidon acétylé.

La suspension d'amidon est préparée à une matière sèche comprise entre 30 et 40% en poids de manière à obtenir une suspension dans l'eau.

Cette suspension est ensuite prégélatinisée au moyen d'une tuyère à la sortie de laquelle est appliquée une contre-pression de vapeur suffisante pour atteindre une température de cuisson de la suspension comprise entre 125 et 135°C tout en maintenant une viscosité acceptable de manière à ce que le débit du lait sortant après cuisson ne soit pas trop faible. Par exemple, on appliquera des contre-pressions de l'ordre de 2 à 3 bars.

De façon surprenante et inattendue, cette température de cuisson est nécessaire et suffisante pour obtenir une bonne solubilité de l'amidon prégélatinisé. La fraction amylose se trouve, selon le procédé conforme à l'invention, sous forme amorphe, alors que les procédés de l'art antérieur de cuisson d'amidons riches en amylose conduisent à une amylose qui a une forte tendance à rétrograder.

On entend par tuyère tout dispositif de cuisson directe par jet de vapeur permettant la cuisson instantanée d'une suspension d'amidon au moyen de vapeur d'eau sous pression.

On obtient à l'issue de cette cuisson une colle d'amidon prégélatinisé qui est ensuite séchée sur tambour sécheur.

Un tel équipement permet de reproduire sur un seul et même dispositif les étapes de cuisson et de séchage en exploitant la chaleur transférée depuis la surface des tambours chauffés à la vapeur jusqu'à la colle d'amidon. On appliquera par exemple, suivant le type d'amidon utilisé, des pressions de vapeur de l'ordre de 2 à 10 bars. La colle d'amidon est étalée uniformément en une mince pellicule sur la surface chaude des tambours par des éléments applicateurs. Les tambours sont animés d'un mouvement de rotation, à une vitesse de l'ordre de 5 à 8 tours par minute. Ainsi, la cuisson de la colle se poursuit, suivie du séchage. Le film formé est ensuite raclé à l'aide d'un couteau racleur de manière à décoller une feuille qui peut être ensuite broyée.

Le procédé selon l'invention permet d'obtenir des amidons riches en amylose prégélatinisés de façon homogène sans qu'il soit nécessaire d'avoir recours à de très hautes températures comme l'enseigne l'art antérieur. Le procédé selon l'invention est particulièrement avantageux par le fait qu'il met en oeuvre des techniques simples, avec un coût en énergie plus faible que les techniques connues d'atomisation ou d'autoclavage.

Les amidons riches en amylose prégélatinisés selon l'invention sont caractérisés par une densité apparente inférieure à 0,4 g/ml pour une granulométrie moyenne de 100 micromètres, ce qui est particulièrement surprenant compte tenu des densités plus élevées obtenues selon les techniques de l'art antérieur et notamment celles décrites dans le brevet EP-B1 0.366.898.

Leur solubilité dans l'eau tiède est bonne, voisine de 80%, alors que celle-ci est inférieure à 30% lorsqu'on supprime l'étape de cuisson sur tuyère.

Ces amidons présentent en outre différents avantages applicatifs comme notamment une bonne capacité à la compression, et surtout de très bonnes aptitudes à former un film. Cette aptitude est particulièrement intéressante pour le pelliculage de comprimés notamment mais aussi pour de nombreuses autres applications nécessitant la formation d'une barrière à l'humidité et à l'air. A la connaissance de la Demanderesse, il n'existait pas sur le marché de tels amidons riches en amylose prégélatinisés susceptibles d'avoir ces propriétés filmogènes. L'invention a donc en outre pour objet l'utilisation d'amidons riches en amylose prégélatinisés pour la fabrication de capsules molles ou de gélules, ainsi que pour l'enrobage de formes solides en pharmacie, alimentation humaine, alimentation animale, agrochimie ou encore de substances végétales telles que les semences.

L'invention a également pour objet l'utilisation d'amidons riches en amylose obtenus selon l'invention pour la préparation de films rafraîchissants, encore appelés feuilles d'arômes. Ces feuilles d'arômes sont des petits carrés de films alimentaires, très fins, fortement aromatisés, utilisés comme rafraîchisseurs d'haleine par dissolution rapide dans la cavité buccale. La demanderesse a constaté qu'en plus des propriétés filmogènes remarquables des amidons selon l'invention, ceux-ci présentaient une aptitude avantageuse à encapsuler les molécules de faible solubilité dans l'eau comme les arômes ou certains principes actifs. En effet, lors de la formulation de tels films à base de polymères filmogènes, lorsqu'on ajoute environ 5% d'arôme ou de principe actif peu solubles dans l'eau à la préparation filmogène, il apparaît lors de l'étalement du film un déphasage total entre les molécules hydrophobes et les autres composants. La solution filmogène est repoussée sur les côtés de la bande de film, alors que le centre n'est composé que d'arôme ou de principe actif. Le film obtenu ne constitue donc pas une pellicule homogène. L'ajout, avant incorporation de l'arôme, d'un amidon riche en amylose prégélatinisé selon l'invention permet, de manière surprenante et inattendue, une inclusion des arômes ou principes actifs peu solubles dans l'eau d'une part, et une excellente qualité de film d'autre part. Sans vouloir se lier à une quelconque théorie, la Demanderesse est d'avis que cette propriété d'encapsulation est liée à l'état amorphe de l'amylose précuite selon le procédé conforme à l'invention, étant donné que les amidons riches en amylose précuits selon les procédés de l'art antérieur ne possèdent pas cette propriété d'encapsulation. Le film obtenu présente les propriétés recherchées, à savoir une bonne solubilité, des propriétés mécaniques suffisantes, une forte aromatisation et pas d'adhérence entre chaque film lors du conditionnement.

Ainsi, un film alimentaire fortement aromatisé ou comprenant un principe actif peu soluble dans l'eau, caractérisé en ce qu'il comprend un amidon riche en amylose prégélatinisé selon l'invention, constitue à la connaissance de la Demanderesse un nouveau produit industriel. On entend par « peu solubles dans l'eau », selon la présente invention, des arômes ou principes actifs qui présentent une solubilité dans l'eau inférieure à 5% en poids.

Selon un mode de réalisation préférentiel du film selon l'invention, ce dernier comprend sur matière sèche 5 à 40%, de préférence 5 à 25% d'amidon riche en amylose prégélatinisé selon l'invention. Selon un mode de réalisation de l'invention encore plus préférentiel, ledit amidon riche en amylose prégélatinisé est stabilisé, par exemple par acétylation ou hydroxypropylation.

Le film comprend en outre tout autre polymère filmogène couramment utilisé dans la pratique, comme notamment les amidons riches en amylopectine, modifiés ou non, les amidons riches en amylose, modifiés ou non, les maltodextrines standard ou spéciales, les polysaccharides et les protéines filmogènes tels que la gélatine, le pullulan, les gommes végétales, le gluten.

Selon une variante préférée, le film alimentaire comprend 5 à 40% d'amidon riche en amylose prégélatinisé selon l'invention et 40 à 80% d'amidon riche en amylopectine. Le complément à 100% est constitué par des arômes, des principes actifs, des édulcorants, un ou plusieurs humectants et éventuellement des colorants. La teneur en eau finale des films selon l'invention est généralement d'environ 12%.

On entend par amidon riche en amylopectine les amidons dits cireux ou waxy, dérivés notamment du maïs ou de la pomme de terre.

Selon une variante encore plus préférentielle, ledit film comprend sur matière sèche 5 à 40%, de préférence 5 à 25%, d'amidon riche en amylose prégélatinisé selon l'invention, et 40 à 80%, de préférence 40 à 70%, d'amidon waxy fluidifié par voie acide.

On choisira de préférence un amidon waxy issu du maïs fluidifié par les techniques connues de l'homme de l'art.

Bien entendu, le film selon l'invention peut être utilisé tel quel en tant que feuille d'arôme pour rafraîchir l'haleine, mais il peut également être utilisé pour le pelliculage de divers supports, notamment alimentaires ou pharmaceutiques. On peut citer sans que ce soit limitatif le pelliculage de comprimés, de confiseries, ou de tout autre support.

Les propriétés avantageuses d'encapsulation de substances peu solubles dans l'eau des amidons selon l'invention peuvent également être mises à profit dans tout autre type d'application nécessitant de telles propriétés, en milieu aqueux ou à l'état sec.

L'invention sera mieux comprise à la lecture des exemples qui suivent, qui se veulent explicatifs et non limitatifs.

### Exemple 1

On prépare des amidons riches en amylose modifiés ou non selon un procédé comprenant les étapes suivantes :
- préparation d'une suspension d'amidon et d'eau
- cuisson sur tuyère
- séchage sur tambour sécheur
- récupération d'une poudre

Dans tous les cas, l'amidon de départ est l'EURYLON® 7 commercialisé par la Demanderesse, présentant une teneur en amylose de 70% en poids.

Les différents paramètres du procédé sont repris dans le tableau 1, ainsi que les densités apparentes et les granulométries moyennes des différents produits obtenus. La densité apparente est mesurée selon la méthode de pharmacotechnie 2.9.16 de la Pharmacopée Européenne, 3^{ème} édition. La granulométrie moyenne est calculée à partir de la granulométrie mesurée par tamisage sur tamis successifs de 500, 315, 200, 150, 100 et 50 micromètres.

La densité de déplacement est mesurée selon la méthode décrite dans le brevet EP-B1 0.366.898.

| | **ESSAI 1 EURYLON**^{**®**}**7** | **ESSAI 2 EURYLON**^{**®**}**7 ACETYLE** | **ESSAI 3 EURYLON**^{**®**}**7 ACETYLE** | **ESSAI 4 EURYLON**^{**®**}**7 OCTENYL SUCCINATE** | **ESSAI 5 EURYLON**^{**®**}**7 HYDROXYPROPYLE** | **ESSAI 6 EURYLON**^{**®**}**7 FLUIDIFIE ACIDE** |
|---|---|---|---|---|---|---|
| **MATIERE SECHE (%)** | 33,5 | 33,4 | 33,4 | 38 | 35,4 | 39 |
| **TEMPERATURE TUYERE (°C)** | 130 | 135 | 135 | 133 | 135 | 135 |
| **CONTRE PRESSION TUYERE (Bars)** | 2,67 | 3,1 | 3,1 | 2,92 | 3,1 | 3,1 |
| **VITESSE DE ROTATION TAMBOUR(tours/min)** | 6,5 | 7 | 8 | 6,5 | 7 | 6,5 |
| **PRESSION TAMBOUR (Bars)** | 10 | 10 | 2 | 10 | 10 | 10 |
| **GRANULOMETRIE MOYENNE (µm)** | 88,2 | 101,2 | 100,8 | 100,6 | 100,6 | 100,4 |
| **DENSITE APPARENTE** (g/ml) | 0,31 | 0,22 | 0,23 | 0,34 | 0,16 | 0,37 |
| **DENSITE DE DEPLACEMENT (g/ml)** | 1,26 | 1,26 | 1,2 | 1,6 | 1,05 | 1,95 |

### Exemple 2

Afin d'en illustrer les propriétés filmogènes, le produit obtenu selon l'essai n°3 de l'exemple précédent est solubilisé dans de l'eau chaude (70-80°C) sous agitation mécanique, à 15% de matière sèche.

La viscosité Brookfield est de 4000 mPa s à 70°C et de 5000 mPa s à 60°C.

Cette solution est déposée sur un support de polyéthylène sur une épaisseur de 45,7 micromètres.

La solution ainsi déposée est séchée à température ambiante, et donne un film rigide, non cassant, et insoluble dans l'eau.

Ce film peut donner lieu à de très nombreuses applications, puisqu'il peut servir à revêtir tous types de formes solides que l'on souhaite protéger, qu'elle soient pharmaceutiques (comprimés, gélules), alimentaires (frites, fonds de tartes, confiseries) ou agricoles (semences, granulés).

Ces propriétés filmogènes peuvent également être mises en application dans la fabrication de capsules molles ou de gélules.

### Exemple 3

On prépare des feuilles d'arôme selon le mode opératoire suivant :

### Formule

| | **Composition de la suspension (%)** | **Quantité mise en oeuvre (g)** | **Composition du film sur sec (%)** |
|---|---|---|---|
| **Amidon waxy fluidifié (CLEARGUM**^{**®**}**CB90)** | 27,75 | 138,75 | 55,5 |
| **Amidon riche en amylose selon l'essai 2 exemple 1** | 4,60 | 23,00 | 9,2 |
| **Arôme menthe SILESIA** | 2,50 | 12,50 | 5,0 |
| **Glycérol** | 15,0 | 75,0 | 30,0 |
| **Saccharinate de sodium** | 0,15 | 0,75 | 0,3 |
| **Eau** | 50,0 | 250,0 | - |
| **total** | 100 | 500 | 100 |

- Dans un bol inox à double enveloppe, disperser le CLEARGUM® dans l'eau, le glycérol et le saccharinate. Chauffer à 90°C.
- Transvaser dans le bol d'un mélangeur KENWOOD, ajouter l'amidon riche en amylose prégélatinisé et l'arôme. Mélanger 1 minute à la vitesse minimale (solution S)
- Transvaser la solution S dans le bol inox et chauffer à 85°C, désaérer au maximum le mélange.
- Lorsque le mélange est à 85°C, former un film sur plaque plastique (épaisseur de colle = 1,5mm) avec un applicateur de film automatique (bar-coater B-2105, RK printcoat)équipé d'une réglette préchauffée à 60°C.
- Laisser sécher en étuve à 20°C et 80% d'humidité relative pendant 2 jours puis terminer le séchage à 20°C en atmosphère non contrôlée.

Le film ainsi obtenu, fortement aromatisé, est très soluble, souple et flexible, non cassant et non fissuré, régulier et homogène en ce qui concerne la distribution de l'arôme, transparent. Il ne colle pas au contact d'autres films lors d'un stockage prolongé. Il peut donc aisément être utilisé comme feuille d'arôme rafraîchissante, ou pour envelopper ou enrober tout type de support notamment alimentaire ou pharmaceutique.

Un film préparé de la même façon mais sans incorporer d'amidon selon l'invention est totalement hétérogène en son centre et impossible à décoller de son support sous forme d'une feuille uniforme.

## Revendications

1. Procédé de préparation d'amidon riche en amylose prégélatinisé **caractérisé en ce qu'**il comprend les étapes consistant :
- à former une suspension comprenant un amidon présentant une teneur en amylose supérieure ou égale à 50% en poids et de l'eau ;
- à soumettre ladite suspension à une cuisson par jet de vapeur à une température comprise entre 125 et 135°C de manière à obtenir une colle d'amidon riche en amylose prégélatinisé ;
- à sécher ladite colle d'amidon prégélatinisé sur tambour sécheur à une pression comprise entre 2 et 10 bars de manière à obtenir un amidon riche en amylose prégélatinisé.

2. Procédé selon la revendication 1, **caractérisé en ce qu'**il comprend en outre une étape de broyage de l'amidon riche en amylose prégélatinisé, de manière à obtenir une poudre.

3. Procédé selon l'une ou l'autre des revendications 1 et 2, **caractérisé en ce que** la teneur en amylose dudit amidon est supérieure ou égale à 50% en poids, et de préférence comprise entre 50 et 80% en poids.

4. Procédé selon l'une quelconque des revendications 1 à 3, **caractérisé en ce que** ledit amidon est un amidon de mais.

5. Procédé selon l'une quelconque des revendications 1 à 4, **caractérisé en ce que** ladite suspension présente une matière sèche supérieure à 25% en poids et de préférence comprise entre 30 et 40% en poids.

6. Procédé selon l'une quelconque des revendications 1 à 5, **caractérisé en ce que** ledit amidon riche en amylose est modifié par voie chimique ou physique.

7. Procédé selon la revendication 6, **caractérisé en ce que** ledit amidon modifié est choisi dans le groupe constitué par les amidons acétylés, hydroxypropylés, carboxyméthylés, fluidifiés, et les octényl succinates d'amidon.

8. Procédé selon la revendication 6, **caractérisé en ce que** ledit amidon modifié est un amidon acétylé.

## Claims

1. A process of preparing progelatinized high amylose starch, **characterized in that** it comprises the steps consisting of :
- forming a suspension comprising an amylose starch having an amylose content greater than or equal to 50% by weight and water ;
- subjecting the said suspension to steam jet cooking at a temperature of between 125°C and 135°C so as to obtain a pregelatinized high amylose starch paste ;
- drying said pregelatinized starch paste on a drum dryer at a pressure of between 2 bar and 10 bar, so as to obtain a pregelatinized high amylose starch.

2. The process according to claim 1, **characterized in that** it comprises, in addition, a step of grinding pregelatinized high amylose starch, so as to obtain a powder.

3. The process according to any of claims 1 and 2, **characterized in that** the amylose content of said starch is greater than or equal to 50% by weight, and preferably of between 50 and 80% by weight.

4. The process according to any of claims 1 to 3, **characterized in that** said starch is a maize starch.

5. The process according to any of claims 1 to 4, **characterized in that** said suspension has a dry matter content greater than 25% by weight and preferably of between 30 and 40% by weight.

6. The process according to any of claims 1 to 5, **characterized in that** said high amylose starch is chemically or physically modified.

7. The process of claim 6, **characterized in that** said modified starch is selected from the group consisting of acetylated, hydroxypropylated, carboxymethylated and fluidified starches, and starch actenyl succinates.

8. The process of claim 6, **characterized in that** said modified starch is an acetylated starch.

## Patentansprüche

1. Verfahren zur Herstellung von vorgelatinisierter amylosereicher Stärke, **dadurch gekennzeichnet, dass** es folgende Schritte umfasst:
Bildung einer Suspension, die eine Stärke mit einem Gehalt an Amylose oberhalb von oder gleich 50 Gew.-% und Wasser umfasst;
Kochen der Suspension mit Dampfstrahl bei einer Temperatur im Bereich zwischen 125 und 135°C, um einen Leim aus vorgelatinisierter amylosereicher Stärke zu erhalten;
Trocknung des Leimes aus vorgelatinisierter Stärke auf einer Trockentrommel bei einem Druck im Bereich zwischen 2 und 10 bar, um eine vorgelatinisierte amylosereiche Stärke zu erhalten.

2. Verfahren nach Anspruch 1, **dadurch gekennzeichnet, dass** es ferner einen Mahlschritt der vorgelatinisierten amylosereichen Stärke umfasst, um ein Pulver zu erhalten.

3. Verfahren nach Anspruch 1 oder 2, **dadurch gekennzeichnet, dass** der Gehalt an Amylose der Stärke oberhalb von oder gleich 50 Gew.-% und vorzugsweise zwischen 50 und 80 Gew.-% liegt.

4. Verfahren nach einem der Ansprüche 1 bis 3, **dadurch gekennzeichnet, dass** die Stärke eine Maisstärke ist.

5. Verfahren nach einem der Ansprüche 1 bis 4, **dadurch gekennzeichnet, dass** die Suspension eine Trockenmasse oberhalb von 25 Gew.-% und vorzugsweise zwischen 30 und 40 Gew.-% enthält.

6. Verfahren nach einem der Ansprüche 1 bis 5, **dadurch gekennzeichnet, dass** die amylosereiche Stärke chemisch oder physikalisch modifiziert ist.

7. Verfahren nach Anspruch 6, **dadurch gekennzeichnet, dass** die modifizierte Stärke ausgewählt ist aus der Gruppe bestehend aus acetylierten Stärken, hydroxypropylierten Stärken, carboxymethylierten Stärken, fluidisierten Stärken und Stärke-Octenylsuccinaten.

8. Verfahren nach Anspruch 6, **dadurch gekennzeichnet, dass** die modifizierte Stärke eine acetylierte Stärke ist.
